(19) Europäisches Patentamt
European Patent Office
Office européen des brevets

(11) EP 4 549 621 A1

(12) EUROPEAN PATENT APPLICATION

(43) Date of publication:
**07.05.2025 Bulletin 2025/19**

(21) Application number: **24207994.5**

(22) Date of filing: **22.10.2024**

(51) International Patent Classification (IPC):
***C25B 1/04*** *(2021.01)* ***C25B 15/08*** *(2006.01)*
***C01B 4/00*** *(2006.01)* ***C01C 1/04*** *(2006.01)*
***C07B 59/00*** *(2006.01)* ***C07C 209/14*** *(2006.01)*

(52) Cooperative Patent Classification (CPC):
**C25B 1/04; C01B 4/00; C01C 1/0405; C07B 59/001; C07C 209/14; C25B 15/081;**
C07B 2200/05

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 EP 23207005**

(71) Applicant: **BASF SE**
**67056 Ludwigshafen am Rhein (DE)**

(72) Inventors:
- **Vossen, Marcus**
  **67117 Limburgerhof (DE)**
- **Ernst, Martin**
  **67056 Ludwigshafen am Rhein (DE)**
- **Stock, Christoph**
  **67056 Ludwigshafen am Rhein (DE)**
- **Hueffer, Stephan**
  **67063 Ludwigshafen (DE)**
- **Weiss, Thomas**
  **67056 Ludwigshafen am Rhein (DE)**
- **Krueger, Marco**
  **67056 Ludwigshafen am Rhein (DE)**
- **Xiong, Jiawen**
  **67056 Ludwigshafen am Rhein (DE)**
- **Harhausen, Sina**
  **67056 Ludwigshafen am Rhein (DE)**

(74) Representative: **BASF IP Association**
**BASF SE**
**GBI - Z078**
**67056 Ludwigshafen (DE)**

# (54) PROCESS FOR MAKING AMINES FROM ALCOHOLS USING HYDROGEN HAVING LOW DEUTERIUM CONTENT PRODUCED WITH NON-FOSSIL ENERGY

(57) A process for the preparation of amines comprising the following steps:
(a) providing hydrogen with a molar share of deuterium ≤ 100 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with an alcohol R-OH in the presence of hydrogen from step (a) to form the corresponding primary, secondary and/or tertiary amines $R-NH_2$, $R_2NH$ and/or $R_3N$.

EP 4 549 621 A1

## Description

The present invention relates to a process for making amines having a lower deuterium content, based on the total hydrogen content, compared to amines generated by reaction with hydrogen produced from petrochemical processes using natural gas, condensate or petroleum oil, the amines obtained thereby as well as to their use. More specifically, the present invention relates to a process for making amines from alcohols using hydrogen having low deuterium content produced from non-fossil electrical energy, the amines obtained thereby as well as to the use of the molar share of deuterium in the hydrogen bound in the amines for tracing the origin of the hydrogen.

In the preparation of amines from alcohols, ammonia is a key precursor. Since the development of the Haber-Bosch process for the preparation of ammonia, the vast majority of ammonia is manufactured by the direct synthesis from hydrogen and nitrogen in the presence of a catalyst, especially an iron-containing catalyst. Special care needs to be taken with the provision of the starting materials hydrogen and nitrogen. They should exhibit a high purity and be substantially free from catalyst poisoning agents such as carbon monoxide and sulfur compounds such as $H_2S$ and $SO_2$. In modern processes, a significant amount of the hydrogen is provided by steam reforming, thus, from natural gas.

However, the petrochemical steam reforming process has its negative impacts with regard to its carbon footprint including the consumption of a lot of fossil-based natural resources and energy.

It is therefore an object of the present invention to provide environmentally friendly amines in an environmentally friendly process for making the same, that process using as little fossil-based energy as possible.

The object is achieved by a process for the preparation of amines, wherein said process comprises the following steps:

(a) providing hydrogen with a molar share of deuterium $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content, by electrolysis of water using electrical power generated at least in part from non-fossil energy,

(b) reacting the hydrogen from step (a) with nitrogen to form ammonia,

(c) reacting the ammonia from step (b) with an alcohol R-OH in the presence of hydrogen from step (a) to form the corresponding primary, secondary and/or tertiary amines $R-NH_2$, $R_2NH$ and/or $R_3N$.

The alcohol used in step c) can be a primary alcohol a secondary alcohol or a tertiary alcohol.

The alcohols used in step c) can be mono-alcohols, diols or triols as well as polyols

Diols can form cyclic structures, see for example formula I and IV below.

The reaction in step c) can be done a mixture of different alcohols (R'-OH, R"-OH, R‴-OH), creating amines $R-NH_2$, $R_2NH$ and/or $R_3N$ with any R = R', R" and/or R‴ being independent of each other. Generally, the R groups can be equal or different from each other. Exemplary amine structures are formula (I) to (IV):

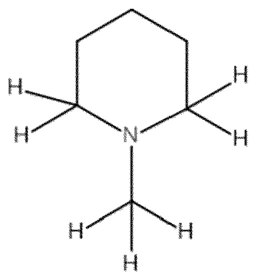

Formula I

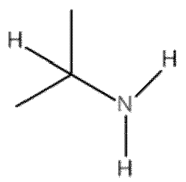

Formula II

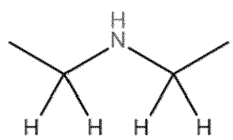

Formula III

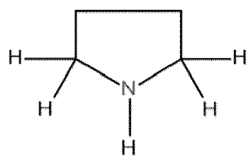

**Formula IV**

In a further embodiment of the present invention, the object is achieved by amines, wherein the molar share of

deuterium in the hydrogen bound on the amine nitrogen and on carbon atoms adjacent (i. e., in alpha-position) to that amine nitrogen in the primary, secondary or tertiary amine group or groups formed in step (c) is ≤ 100 ppm, preferably in the range of from 10 to 95 ppm, more preferably in the range of from 15 to 90 ppm, most preferably in the range of from 20 to 80 ppm, especially in the range of from 30 to 75 ppm based on the total hydrogen content based on the total hydrogen content bound on the amine nitrogen and on carbon atoms adjacent to that amine nitrogen.

It is important that the origin of the hydrogen and downstream compounds obtained by clean energy can be tracked in a reliable way. This is especially important to ensure that:

- Hydrogen and downstream compounds have been produced in accordance with sustainability criteria.
- Renewable attributes aren't subject to double counting.

Companies are placing increasing importance on sourcing green energy. Because of this, tracking systems have to be developed for the origin of the energy used in the preparation of hydrogen and downstream compounds.

This object is also achieved by use of the molar share of deuterium in hydrogen bound in downstream compounds based on hydrogen for tracing the origin, especially the energetic origin, of the hydrogen bound in the downstream compounds based on hydrogen, wherein the compounds are amines and a process for tracing the origin, especially the energetic origin, of hydrogen bound in downstream compounds based on hydrogen by determining the molar share of deuterium in the hydrogen bound in said downstream compounds. Methods for determination of the molar share of deuterium in hydrogen and in downstream compounds based on hydrogen are known to a person skilled in the art and include mass spectrometry and NMR technologies.

A further environmental benefit of the environmentally friendly amines according to the present invention is their use in carbon capturing processes, since the amines according to the present invention are produced using as little fossil-based energy as possible, ideally no fossil-based energy, and do therefore only add as little as possible, ideally nothing, to $CO_2$ emission.

A further embodiment of the present invention is therefore the use of the amines according to the present invention as liquid or solid $CO_2$ absorbents in $CO_2$ capturing processes. Specific examples of amines usable with preference are:

i) 2-aminoethoxyethanol (AEE or ADEG), 2-aminoethanol (monoethanolamine), 2-(methylamino)ethanol, 2-(ethylamino)ethanol, 2-(n-butylamino)ethanol, 2-amino-2-methylpropanol, N-(2-aminoethyl)piperazine, methyldiethanolamine, ethyldiethanolamine, dimethylaminopropanol, tert-butylaminoethoxyethanol (TBAEE), methoxyethoxyethyl-tert-butylamine, 2-amino-2-methylpropanol, diisopropanolamine (DIPA or DIPOA);

ii) 3-methylaminopropylamine, ethylenediamine, diethylenetriamine, triethylenetetramine, 2,2-dimethyl-1,3-diaminopropane, hexamethylenediamine, 1,4-diaminobutane, 3,3-iminobispropylamine, tris(2-aminoethyl)amine, bis(3-dimethylaminopropyl)amine, tetramethylhexamethylenediamine;

iii) piperazine, 2-methylpiperazine, N-methylpiperazine, 1-hydroxyethylpiperazine, 1,4-bishydroxyethylpiperazine, 4-hydroxyethylpiperidine, homopiperazine, piperidine, 2-hydroxyethylpiperidine, triethylenediamine (TEDA) and morpholine; and

iv) mixtures thereof.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1H$ and deuterium $^2H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of

a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general $\leq$ 100 ppm, preferably in general $\leq$ 90 ppm, for example from 30 to 75 ppm. The deuterium atom content in electrolytically produced hydrogen may be as low as 10 ppm. The deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 mA/$cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nafion®, fumapem®) are used as an electrolyte (proton conductor). These proton exchange membranes have many advantages such as low gas permeability, high proton conductivity ($0.1 \pm 0.02$ S $cm^{-1}$), low thickness (20-300 $\mu$m), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2/RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 $\mu$m) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials

are embedded in a polymer matrix. One of the most common and commercially available PEM materials is the fluoropolymer PFSA, or Nation®, a DuPont product. While Nation® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 -2133.

K. Harada et al., International Journal of Hydrogen Energy 45 (2020), pp. 31389 - 31 395 report a deuterium depletion by a factor from 2 to 3 in polymer electrolyte membrane water electrolysis. The separation factor β

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A $cm^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), ρ is the density of water (g $mL^{-1}$) and $M_{H2O}$ (g $mol^{-1}$) is the molar weight of water. A stoichiometric number A of 10 means that 10 times the amount of fresh water than can be theoretically consumed by electrolysis at the given electrolysis current is supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%.

The electrical power from non-fossil resources used in water electrolysis according to the invention can be generated by nuclear energy. Nuclear energy is considered renewable by the European Commission, as long as certain preconditions (inter alia safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary. Dry steam is the oldest form of geothermal technology and

takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

Step (b) concerns the reaction of the hydrogen from step (a) with nitrogen to form ammonia.

The reaction of step (b) preferably follows the Haber-Bosch process.

The catalysts usually used in the Haber-Bosch process generally fall into one of two categories, fused-iron and supported metallic catalysts. Fused-iron catalysts are derived from iron oxides, of which there are three possibilities: $Fe_2O_3$, $Fe_3O_4$, and $Fe_{1-x}O$, which are known as hematite, magnetite, and wüstite, respectively. Industrially, these iron catalysts will be multipromoted with promoters such as $K_2O$, BaO, KOH, CaO, MgO and $Al_2O_3$ are present in small quantities of a few weight percent. Supported metallic catalysts are catalysts made up of a metallic catalyst material, normally ruthenium or cobalt for the ammonia synthesis reaction, present on the surface of a support material, normally activated carbon or a metal oxide. Commonly the weight percentage of the metallic catalyst is around 2-10%.

Other catalysts which may be used are nickel, and nitride catalyst systems or hydride, nitride, oxynitride hydride promoted Ru, Fe, Co, and Ni catalysts.

The catalysts mentioned above can be used for both conventional centralized large-scale Haber-Bosch ammonia synthesis plants and distributed small-scale ammonia production via the same process.

In one embodiment of the present invention, step (b) is performed at a pressure in the range of from 50 to 350 bar (abs), preferably 150 to 300 bar (abs).

In one embodiment of the present invention, step (b) is performed at a temperature in the range of from 300 to 600 °C, preferably 400 to 500 °C.

By performing step (b), ammonia is formed. The deuterium content of the ammonia is significantly lower than obtained by classical petrochemical routes. The deuterium content is in general $\leq$ 100 ppm, preferably in the range of from 10 to 95 ppm, more preferably in the range of from 15 to 90 ppm, most preferably in the range of from 20 to 80 ppm, especially in the range of from 30 to 75 ppm, based on the total hydrogen content.

The overall kinetic isotope effect is cumulative, since it will also be present in all subsequent production steps downstream the value chain.

Step (c)

In step (c), the ammonia from step (b) is reacted with an alcohol R-OH in the presence of hydrogen from step (a) to

form the corresponding primary, secondary and/or tertiary amines $R-NH_2$, $R_2NH$ and/or $R_3N$.

The alcohol used in step c) can be a primary alcohol, a secondary alcohol or a tertiary alcohol. The alcohols used in step c) can be mono-alcohols, diols or triols as well as polyols.

Diols can form cyclic structures (for example see formula I and IV)

The reaction in step c) can be done with mixtures of different alcohols (R'-OH, R"-OH, R‴-OH), creating amines $R-NH_2$, $R_2NH$ and/or $R_3N$ with any R = R', R" and/or R‴ being independent of each other (see formula I for example).

Generally, the R groups can be equal or different from each other.

The process of converting alcohols into the corresponding amines can be carried out in various ways, which can be differentiated according to the type of catalyst, the feedstock and the physical state of the reactants.

The process can be catalyzed heterogeneously or homogeneously.

Among the heterogeneous catalysts, supported metal catalysts and solid acid catalysts are mainly used, according to the type of alcohol used as feedstock. Methanol is converted to methyl amines over solid acid catalysts in the absence of hydrogen while higher alcohols are reacted over transition metal containing catalysts in the presence of hydrogen in a so-called reductive amination (a better term would be amination under reducing conditions as there is no net reduction in the process in contrast to the reaction of carbonyls with amines or ammonia to give amines which is also called reductive amination) via borrowing hydrogen or hydrogen autotransfer mechanism. A review on the industrially relevant catalytic systems and technologies is given by K.S. Hayes, in Applied Catalysis A: General 221 (2001) 187-195. General information on the conversion of alcohols to amines can also be found i' Ullmann's Encyclopedia of Industrial Chemistry, "Amines, Aliphatic", pp 6-7.

The main catalytically active metals employed in the heterogeneously catalyzed amination of alcohols are Ni, Co and Cu, with the latter as well as additional noble metals (Rh, Ir, Pd, Pt, Ag, Au) serving to enhance reducibility or activity of the main catalyst components. Other metals (Fe, Mn, Ru, Re, Mo, Sn, La) are frequently admixed to moderate by-product formation or increase the selectivity. The current state of the art for Ni and Co-based catalysts has been reviewed by C. Yue et al., in the Arabian Journal of Chemistry 2022, 15, 103865.

The processes can be further divided into liquid and vapor phase processes with notable differences in the reaction pressure. Low boiling alcohols are frequently aminated in the vapor phase. Among the liquid phase processes, the operational mode can be continuous, fed-batch (also called semi-continuous) or batch. Aromatic alcohols (phenols) are an exception as they are aminated with concomitant hydrogenation of the aromatic core and re-aromatized in the process. In this case, heterogeneous catalysts which display aromatics hydrogenation activity, mainly comprising Pd, Pt and Ru, are used. The reaction can take place in the liquid or gas phase.

While the heterogeneously catalyzed amination of alcohols is known since the early 1900 and carried out on technical scale since the 1940s, the homogeneously catalyzed amination of alcohols with ammonia has emerged over the last 30 years. In many cases, the reaction conditions are milder, or the reaction proceeds with higher selectivity towards the desired products, e.g., primary amines when secondary or tertiary amines can be formed in consecutive reactions. Biobased feedstocks have been successfully aminated as well. The technology has been reviewed by B. Morandi et al. in Synthesis 2017, 49, 776-789, Q. Yang et al. in Chem. Soc. Rev. 2015, 44, 2305-2329 and S. Bähn et al. in ChemCatChem 2011, 3, 1853-1864. The catalysts vary broadly but mostly comprise complexes of a transition metals like Ru, Ir, Rh, Fe, Co and Mn with phosphine or cyclopentadienyl ligands. Well-known phosphine ligand architectures include acridines, diphenylethers and xanthenes (Xantphos) and alkylideneamines (dppe, dppp) as well as alkanes (triphos). Specific alcohols require differentiated catalysts for optimum results as described in WO 2014/016241, WO2012/119927 and WO2012/119929 (all to BASF) or WO2012/113475 (Evonik).

In certain embodiments, the amination of alcohols with ammonia in the presence of hydrogen accordingto step (c) of the process of the invention is carried out as heterogeneously catalyzed amination.

The heterogeneously catalyzed amination can be performed continuously or batchwise. Preference is given to a continuous method. The catalyst is composed of various active metals on an oxidic support, and either extruded or shaped into pellets or other more intricate shaped bodies. The active metals are selected from transition metals, preferably from Ni, Co, Cu, Ag, Au, Pd, Pt, Rh, Ru, Ir, Fe. Optionally, other metals can be added to moderate the activity and selectivity of the catalytically active metals, like Mo, Cr, Zn, Sb, Bi, Sn, Mn. Other elements like rare earth metals, e.g., La or Y, and main group elements like Li, Na, K, Ca, Ba, Cs, P, S, or carbon may also be present and can enhance mechanical stability, selectivity or lifetime of the catalysts. Preferred are Ni, Co, Cu, Ag, Pd, Pt, Ru as active metals, and Sn, Cr, Zn, Mo, Mn, La, Li, Na, P as further admixtures. Supports are chosen from aluminum oxide, silicon oxide, zirconium oxide, titanium oxide, active carbon, preferably from aluminum oxide, silicon oxide, zirconium oxide or mixtures of these metal oxides. Optionally, particles of elemental metals and binders like cement can also be added to the catalyst to facilitate the shaping or prolonging the lifetime.

For the synthesis in the gas phase, the reactants are fed to the reactor in a controlled manner, preferably in a cycle gas stream, evaporated and in gaseous form. Suitable amines for a gas phase synthesis are amines which, owing to their boiling points and the boiling points of their reactants, can be kept in the gas phase within the process parameters by process technology means. Examples are the amination of ethanol, propanol, isopropanol, butanol, methoxyisopropanol,

diethylene glycol, phenol, dimethylphenol, butanediol, pentanediol. The cycle gas serves firstly to evaporate the reactants and secondly as a reactant for the amination. In the cycle gas method, the starting materials (alcohol, hydrogen and ammonia) are evaporated in a cycle gas stream and fed to the reactor in gaseous form. The reactants (alcohol and ammonia) may also be evaporated as aqueous solutions and passed into the catalyst bed with the cycle gas stream. Preferred reactors are tubular reactors. Examples of suitable reactors with cycle gas stream can be found in Ullmann's Encyclopedia of Industrial Chemistry, 5th Ed., Vol. B 4, pages 199-238, "Fixed-Bed Reactors". Alternatively, the reaction is advantageously carried out in a tube bundle reactor or in a single-stream plant. In a single-stream plant, the tubular reactor in which the reaction proceeds can consist of a connection of a plurality of (e.g., two or three) individual tubular reactors in series. Optionally, an intermediate introduction of feed (comprising the reactant and/or ammonia and/or $H_2$) and/or cycle gas and/or reactor effluent from a downstream reactor is possible here. The cycle gas flow rate is preferably in the range from 40 to 1500 $m^3$ (at operating pressure)/[$m^3$ of catalyst (bed volume) x h], in particular in the range from 100 to 700 $m^3$ (at operating pressure)/[$m^3$ of catalyst (bed volume) x h]. The cycle gas comprises preferably at least 10% by volume, particularly from 20 to 100% by volume, very particularly from 50 to 100% by volume of $H_2$. Flow through the fixed bed of catalyst can be either from the top or from the bottom. The temperature, pressure and amount of the gas stream are set so that even relatively high-boiling reaction products remain in the gas phase. Ammonia is preferably used in a from 0.90- to 100-fold molar amount, in particular in a from 1.0- to 10-fold molar amount, in each case based on the alcohol used. As primary amines react faster than ammonia with the starting alcohol, secondary and tertiary amines can be formed consecutively. The proportion of the various amines can be controlled by varying the excess of ammonia. Similarly, whenever there is a possibility for cyclizing, e.g., when several hydroxy groups are present in proximity to each other, the tendency to form a cycle can be controlled by varying the excess of ammonia. A greater excess will lead to more of the open-chain product.

The amination is preferably carried out at an absolute pressure in the range from 1 to 300 bar, preferably from 1 to 50 bar, particularly preferably from 1 to 30 bar. The amination is preferably carried out at a temperature in the range from 80 to 300°C, preferably from 150 to 250°C, particularly preferably from 170 to 230°C. The process is preferably operated with an amount of off-gas of from 5 to 800 standard cubic meters/h, in particular from 20 to 300 standard cubic meters/h. [Standard cubic meters (standard $m^3$)=volume converted to STP]. The space velocity over the catalyst is preferably in the range from 0.1 to 3.0 kg, preferably from 0.1 to 2.0 kg, particularly preferably from 0.2 to 1.5 kg, of alcohol per liter of catalyst (bed volume) and hour. The use of higher temperatures, higher total pressures and higher space velocities over the catalyst is possible. The pressure in the reactor is advantageously increased to the desired reaction pressure by injection of hydrogen. The water of reaction formed during the reaction generally does not have any adverse effect on the conversion, the reaction rate, the selectivity and the operating life of the catalyst and is therefore advantageously removed from the reaction product only in the work-up of the reaction product, e.g., by distillation.

The reaction product mixture is advantageously depressurized and the excess hydrogen and any excess ammonia present are then removed and the crude reaction product obtained is purified, e.g. by means of fractional rectification. Suitable work-up methods are described, for example, in EP 1 312 600 A and EP 1 312 599 A (both BASF AG). Unreacted starting materials and any suitable by-products obtained can be recirculated to the synthesis. After condensation of the products in a separator, unreacted starting materials can once again be passed, in discontinuous or continuous operation, in the circulating gas stream over the catalyst bed.

For the synthesis in the liquid phase, more precisely in a two or three phase process comprising a liquid phase in the reactor, suitable reactants and products are all of those which have high boiling points or are thermally labile. In these cases, a further advantage is that it is possible to dispense with evaporation and recondensation of the amine in the process. Examples are the amination of monoethylene glycol, monoethanolamine, isopropanolamine, octanol, tridecanol and alkoxylation products with molar masses from about 200 to 5000 g/mol. Naturally, a number of alcohols are suitable both for processes in the gas phase and the liquid or mixed phases that cannot be categorized with certainty as one or the other. Some processes might also take place in supercritical phase under the respective process conditions. Ammonia itself becomes supercritical at 132.4 °C, the critical pressure being 113 bar. Whenever ammonia and a well-miscible alcohol are processed with little or no hydrogen at temperatures and pressures above the critical values, the reactants may be present in a supercritical state.

The process can be carried out in continuous mode or in batch- or fed-batch mode. Reactors can be either tubular reactors, continuously stirred tank reactors, recirculating gas lift reactors or combinations thereof. Again, in liquid phase as in the gas phase the process is preferably carried out in continuous mode, and the catalyst is preferably arranged as a fixed bed in the reactor. Flow through the fixed bed of catalyst can be either from the top or from the bottom. In addition to the liquid phase, a gas phase may be present. The reactants (alcohol plus ammonia) are passed simultaneously, including hydrogen, over the catalyst at pressures of generally 5 to 40 MPa (50-400 bar), preferably 5 to 38 MPa, more preferably 15 to 33 MPa, and temperatures of generally 80 to 350°C, particularly 100 to 300°C, preferably 120 to 270°C, more preferably 130 to 250°C, in particular 170 to 230°C. The catalyst hourly space velocity is generally in the range from 0.05 to 5 kg, preferably 0.1 to 2 kg and more preferably 0.2 to 1.5 kg of alcohol per liter of catalyst (bed volume) and hour. If appropriate, the reactants can be diluted with a suitable solvent such as tetrahydrofuran, dioxane, N-methylpyrrolidone or ethylene

glycol dimethyl ether. It is appropriate to heat the reactants before they are fed into the reaction vessel, preferably to the reaction temperature. It is possible to use higher temperatures and higher overall pressures and catalyst hourly space velocities. The pressure in the reaction vessel, which results from the sum of the partial pressures of ammonia, of the alcohol, and of the reaction products formed and, if appropriate, of the solvent used at the temperatures specified, is appropriately increased by injecting hydrogen up to the desired reaction pressure. Excess ammonia can be circulated together with the hydrogen. The water of reaction formed in the course of the reaction (in each case one mole per mole of alcohol group) generally does not have a disruptive effect on the degree of conversion, the reaction rate, the selectivity and the catalyst lifetime, and is therefore appropriately not removed therefrom until the workup of the reaction product, for example by distillation.

After the reaction effluent has appropriately been decompressed, the excess hydrogen and any excess ammonia present are removed therefrom and the resulting crude reaction product is purified, for example by a fractional rectification as described above. Excess ammonia and the hydrogen are advantageously returned into the reaction zone. The same applies to any incompletely converted alcohol. Unconverted reactants and any suitable by-products which are obtained can be returned into the synthesis.

In certain further embodiments, the amination of alcohols with ammonia in the presence of hydrogen according to step (c) of the process of the invention is carried out as homogeneously catalyzed amination.

The homogeneous catalysts can be produced either directly in their active form or only under the reaction conditions from customary precursors with addition of the appropriate ligands. Customary precursors are, for example, [Ru(p-cymene)$Cl_2$]$_2$, [Ru(benzene)$Cl_2$]$_n$, [Ru$(CO)_2Cl_2$]$_n$, [Ru$(CO)_3Cl_2$]$_2$ [Ru(COD)(allyl)], [$RuCl_3$*$H_2O$], [Ru(acetylacetonate)$_3$], [Ru$(DMSO)_4Cl_2$], [Ru$(PPh_3)_3$(CO)(H)Cl], [Ru$(PPh_3)_3$(CO)$Cl_2$], [Ru$(PPh_3)_3$(CO)$(H)_2$], [Ru$(PPh_3)_3Cl_2$], [Ru(cyclopentadienyl)$(PPh_3)_2$Cl], [Ru(cyclopentadienyl)$(CO)_2$Cl], [Ru(cyclopentadienyl)$(CO)_2$H], [Ru(cyclopentadienyl)$(CO)_2$]$_2$, [Ru(pentamethylcyclopentadienyl)$(CO)_2$Cl], [Ru(pentamethylcylcopentadienyl)$(CO)_2$H], [Ru(pentamethylcyclopentadienyl)$(CO)_2$]$_2$, [Ru(indenyl)$(CO)_2$Cl], [Ru(indenyl)$(CO)_2$H], [Ru(indenyl)$(CO)_2$]$_2$, ruthenocene, [Ru(binap)$Cl_2$], [Ru(bipyridine)$_2Cl_2$*$2H_2O$], [Ru(COD)$Cl_2$]$_2$, [Ru(pentamethylcyclopentadienyl)(COD)Cl], [$Ru_3(CO)_{12}$], [Ru(tetraphenylhydroxycyclopentadienyl)$(Co)_2$H], [Ru$(PMe_3)_4(H)_2$], [Ru$(PEt_3)$4$(H)_2$], [Ru$(PnPr_3)_4(H)_2$], [Ru$(PnBu_3)_4(H)_2$], [Ru(PnOctyl$_3$)$_4(H)_2$], [$IrCl_3$*$H_2O$], $KIrC_{l4}$, $K_3IrC_{l6}$, [Ir(COD)Cl]$_2$, [Ir(cyclooctene)$_2$Cl]$_2$, [Ir(ethene)$_2$Cl]$_2$, [Ir(cyclopentadienyl)$Cl_2$]$_2$, [Ir(pentamethylcyclopentadienyl)$Cl_2$]$_2$, [Ir(cylopentadienyl)$(CO)_2$], [Ir(pentamethylcyclopentadienyl)$(CO)_2$], [Ir$(PPh_3)_2$(CO)(H)], [Ir$(PPh_3)$2(CO)(Cl)], [Ir$(PPh_3)_3$(Cl)].

The amount of the metal component of the catalyst, preferably ruthenium or iridium, is generally from 0.1 to 5000 ppm by weight, in each case based on the total liquid reaction medium. The reaction occurs in the liquid phase, generally at a temperature of from 20 to 250°C. The amination according to the invention is preferably carried out at temperatures in the range from 100°C to 200°C, particularly preferably in the range from 110 to 160°C. The reaction can generally be carried out at a total pressure of from 0.1 to 20 MPa absolute, which can be either the autogenous pressure of the solvent at the reaction temperature or the pressure of a gas such as nitrogen, argon or hydrogen. The amination according to the invention is preferably carried out at a total pressure in the range from 0.5 to 10 MPa absolute, particularly preferably at a total pressure in the range from 1 to 6 MPa absolute. The average reaction time is generally from 15 minutes to 100 hours. The aminating agent (ammonia) can be used in stoichiometric, sub-stoichiometric or super-stoichiometric amounts based on the hydroxyl groups to be aminated. If several hydroxy-groups are present, the reaction outcome (mono, di, triamine etc.) can be controlled by the excess of ammonia. In a preferred embodiment, ammonia is used in a from 1- to 250-fold, preferably a from 2- to 100-fold, in particular in a from 1.5- to 10-fold, molar excess per mole of hydroxyl groups to be reacted in the starting material. Higher excesses of ammonia are also possible.

The process of the invention can be carried out either in a solvent or without solvent. Suitable solvents are polar and nonpolar solvents which can be used in pure form or in mixtures. For example, it is possible to use only one nonpolar or one polar solvent in the process of the invention. It is also possible to use mixtures of two or more polar solvents or mixtures of two or more nonpolar solvents or mixtures of one or more polar solvents with one or more nonpolar solvents. The product can also be used as solvent, either in pure form or in mixtures with polar or nonpolar solvents. Suitable nonpolar solvents are, for example, saturated and unsaturated hydrocarbons such as hexane, heptane, octane, cyclohexane, benzene, toluene, xylene and mesitylene and linear and cyclic ethers such as THF, diethyl ether, 1,4-dioxane, MTBE (tert-butyl methyl ether), diglyme and 1,2-dimethoxyethane. Preference is given to using toluene, xylene or mesitylene. Suitable polar solvents are, for example, water, dimethylformamide, formamide, tert-amylalcohol, tert-butanol and acetonitrile. Preference is given to using water. The water can either be added before the reaction, be formed as water of reaction during the reaction or be added after the reaction in addition to the water of reaction. A further preferred solvent is tert-amyl alcohol. Preferred is a mixture of tert-amylalcohol and water.

To carry out the reaction in the liquid phase, ammonia, the alcohol, optionally together with one or more solvents, together with the complex catalyst are introduced into a reactor. The introduction of ammonia, starting material, optionally solvent and complex catalyst can be carried out simultaneously or separately. The reaction can be carried out continuously, in semi-batch mode, in batch mode, admixed in product as solvent or without admixing in a single pass. It is in principle possible to use all reactors which are basically suitable for gas/liquid reactions at the given temperature and

the given pressure for the process of the invention. Suitable standard reactors for gas/liquid reaction systems and for liquid/liquid reaction systems are, for example, indicated in K.D. Henkel, "Reactor Types and Their Industrial Applications", in Ullmann's Encyclopedia of Industrial Chemistry, 2005, Wiley VCH Verlag GmbH & Co. KGaA, DOI: 10.1002/14356007.b04_087, chapter 3.3 "Reactors for gas-liquid reactions". Examples which may be mentioned are stirred tank reactors, tube reactors or bubble column reactors.

The reaction output formed in the reaction generally comprises the corresponding amination products, the one or more solvents if used, the complex catalyst, possibly unreacted starting materials and ammonia and also the water of reaction formed. Any excess ammonia present, any solvent present, the complex catalyst and the water of reaction are removed from the reaction output. The amination product obtained can be worked up further. The excess ammonia, the complex catalyst, any solvent or solvents and any unreacted starting materials can be recirculated to the amination reaction. If the amination reaction is carried out without solvent, the homogeneous complex catalyst is dissolved in the product after the reaction. This can remain in the product or be separated therefrom by a suitable method. Possibilities for separating the catalyst are, for example, scrubbing with a solvent which is not miscible with the product and in which the catalyst dissolves better than in the product as a result of a suitable choice of the ligands. The catalyst concentration in the product is optionally reduced by multistage extraction. As extractant, preference is given to using a solvent which is also suitable for the target reaction, e.g., toluene, benzene, xylenes, alkanes such as hexanes, heptanes and octanes and acyclic or cyclic ethers such as diethyl ether and tetrahydrofuran, which can after concentration by evaporation be reused together with the extracted catalyst for the reaction. It is also possible to remove the catalyst by means of a suitable absorbent. The catalyst can also be separated by adding water to the product phase if the reaction is carried out in a solvent which is immiscible with water. If the catalyst in this case dissolves preferentially in the solvent, it can be removed with the solvent from the aqueous product phase and optionally be reused. This can be brought about by selection of suitable ligands.

The resulting aqueous diamines, triamines or polyamines can be used directly as technical-grade amine solutions. It is also possible to separate the amination product from the catalyst by distillation. If the reaction is carried out in a solvent, the latter can be miscible with the amination product and be separated off by distillation after the reaction. It is also possible to use solvents which have a miscibility gap with the amination products or the starting materials. Suitable solvents for this purpose are, for example, toluene, benzene, xylenes, alkanes such as hexanes, heptanes and octanes and acyclic or cyclic ethers such as diethyl ether, tetrahydrofuran, tert-amylalcohol and dioxane. As a result of suitable choice of the phosphine ligands, the catalyst preferentially dissolves in the solvent phase, i.e., in the phase not comprising product. The phosphine ligands can also be selected so that the catalyst dissolves in the amination product. In this case, the amination product can be separated from the catalyst by distillation. The product may also be used as solvent. The solvent can also be miscible with the starting materials and the product under the reaction conditions and only form a liquid phase comprising the major part of the catalyst after cooling. As solvents which display this property, mention may be made by way of example of toluene, benzene, xylenes, alkanes such as hexanes, heptanes and octanes. The catalyst can then be separated together with the solvent and be reused. The product phase can also be admixed with water in this variant. The proportion of the catalyst comprised in the product can subsequently be separated by means of suitable absorbents such as polyacrylic acid and salts thereof, sulfonated polystyrenes and salts thereof, activated carbons, montmorillonites, bentonites and zeolites or else be left in the product.

The amination reaction can also be carried out in a two-phase system. In the case of the two-phase reaction, suitable nonpolar solvents are, in particular, toluene, benzene, xylenes, alkanes such as hexanes, heptanes and octanes in combination with lipophilic phosphine ligands on the transition metal catalyst, as a result of which the transition metal catalyst accumulates in the nonpolar phase. In this embodiment, in which the product and the water of reaction and any unreacted starting materials form a second phase enriched with these compounds, the major part of the catalyst can be separated from the product phase by simple phase separation and be reused. If volatile by-products or unreacted starting materials or the water formed in the reaction or added after the reaction to aid the extraction are undesirable, they can be separated from the product without problems by distillation. It can also be advantageous for the water formed in the reaction to be removed continuously from the reaction mixture. The water of reaction can be separated from the reaction mixture directly by distillation or as azeotrope with addition of a suitable solvent (entrainer) and using a water separator or be removed by addition of water-withdrawing auxiliaries. The addition of bases can have a positive effect on product formation. Suitable bases which may be mentioned here are alkali metal hydroxides, alkaline earth metal hydroxides, alkaline metal alkoxides, alkaline earth metal alkoxides, alkali metal carbonates and alkaline earth metal carbonates, of 0.01 to 100 molar equivalents, based on the metal catalyst used, can be used.

Suitable alcohols which can be reacted with ammonia in amination step (c) under the above-mentioned prerequisites are virtually all primary and secondary alcohols with an aliphatic function. The alcohols may be straight-chain, branched or cyclic. Secondary alcohols are aminated just as efficiently as primary alcohols. The alcohols may also bear substituents or comprise functional groups which behave inertly under the conditions of the hydrogenating amination, for example alkoxy, alkenyloxy, alkylamino or dialkylamino groups, or else if appropriate are hydrogenated under the conditions of the hydrogenating amination, for example carbon-carbon double or triple bonds. When polyhydric alcohols,

for example, diols or triols, particularly glycols, are to be aminated, it is possible via the control of the reaction conditions to obtain preferentially amino alcohols, cyclic amines or polyaminated products. The amination of 1,2-diols leads, depending on the selection of the reaction conditions, particularly to 1-amino-2-hydroxy compounds or 1,2-diamino compounds. The amination of 1,4-diols leads, depending on the selection of the reaction conditions, to 1-amino-4-hydroxy compounds, 1,4-diamino compounds, or to five-membered rings with a nitrogen atom (pyrrolidines). The amination of 1,6-diols leads, depending on the selection of the reaction conditions, to 1-amino-6-hydroxy compounds, 1,6-diamino compounds, or to seven-membered rings with a nitrogen atom (hexamethyleneimines). The amination of 1,5-diols leads, depending on the selection of the reaction conditions, to 1-amino-5-hydroxy compounds, 1,5-diamino compounds, or to six-membered rings with a nitrogen atom (piperidines, 1,5-dipiperidinylpentanes). It is accordingly possible to obtain from diglycol (DEG), by amination with $NH_3$, monoaminodiglycol (=ADG=$H_2N-CH_2CH_2-O-CH_2CH_2-OH$), diaminodiglycol ($H_2N-CH_2CH2-O-CH_2CH2-NH_2$) or morpholine. Piperazine is correspondingly obtained with from diethanolamine. N-(2-Hydroxyethyl) piperazine can be obtained from triethanolamine.

Preference is given to aminating, for example, the following alcohols: methanol, ethanol, n-propanol, isopropanol, n-butanol, isobutanol, n-pentanol, n-hexanol, 2-ethylhexanol, tridecanol, stearyl alcohol, palmityl alcohol, cyclobutanol, cyclopentanol, cyclohexanol, benzyl alcohol, 2-phenylethanol, 2- (p-methoxyphenyl)-ethanol, 2-(3,4-dimethoxyphenyl) ethanol, 1-phenyl-3-butanol, ethanolamine, n-propanolamine, isopropanolamine, 2-amino-1-propanol, 1-methoxy-2- propanol, 3-amino-2,2-dimethyl-1 -propanol, n-pentanolamine (1-amino-5-pentanol), n-hexanolamine (1-amino-6-hexanol), ethanolamine, diethanolamine, triethanolamine, N-alkyl-diethanolamines, diisopropanolamine, 3-(2-hydroxyethylamino)propan-1-ol, 2-(N,N-dimethylamino)ethanol, 2-(N,N-diethylamino)ethanol, 2-(N,N-di-n-propylamino) ethanol, 2-(N,N-diisopropylamino)ethanol, 2-(N,N-di-n-butylamino)ethanol, 2-(N,N-diisobutylamino) ethanol, 2-(N,N-di-sec-butylamino)ethanol, 2-(N,N-di-tert-butylamino)ethanol, 3-(N,N-dimethylamino)propanol, 3-(N, N-diethylamino) propanol, 3-(N,N-di-n-propylamino) propanol, 3-(N,N-diisopropylamino)propanol, 3-(N,N-di-n-butylamino)propanol, 3-(N,N-diisobutylamino)propanol, 3-(N,N-di-sec-butylamino)-propanol, 3-(N,N-di-tert-butylamino)propanol, 1-dimethylaminopentanol-4,1-diethylaminopentanol-4, ethylene glycol, 1,2-propylene glycol, 1,3-propylene glycol, diglycol, 1,4-butanediol, 1,5-pentanediol, 1,6- hexanediol, 2,2-bis[4-hydroxycyclohexyl]propane, methoxyethanol, propoxyethanol, butoxyethanol, polypropyl alcohols, polyethylene glycol ethers, polypropylene glycol ethers and polybutylene glycol ethers. The latter polyalkylene glycol ethers are converted to the corresponding amines in the inventive reaction by converting their free hydroxyl groups. Examples of diols which can be used as starting materials in the process of the invention are 1,2-ethanediol (ethylene glycol), 1,2-propanediol (1,2-propylene glycol), 1,3-propanediol (1,3-propylene glycol), 1,4-butanediol (1,4-butylene glycol), 1,2-butanediol (1,2-butylene glycol), 2.3-butanediol, 2-methyl-1,3-propanediol, 2,2-dimethyl-1,3-propanediol (neopentyl glycol), 1,5-pentanediol, 1,2-pentanediol, 1,6-hexanediol, 1,2-hexanediol, 1,7-heptanediol, 1,2-heptanediol, 1,8-octanediol, 1,2-octanediol, 1,9-nonanediol, 1,2-nonanediol, 2,4-dimethyl-2,5-hexanediol, the

neopentyl glycol ester of hydroxypivalic acid, diethylene glycol, triethylene glycol, 2-butene-1,4-diol, 2-butyne-1,4-diol, polyethylene glycols, polypropylene glycols such as 1,2-polypropylene glycol and 1,3-polypropylene glycol, polytetrahydrofuran (polytetramethylene glycol), 1,4-bis(2-hydroxyethyl)piperazine, 2,5-(dimethanol)-furan, 1,4-bis(hydroxymethyl)-cyclohexane, N-butyldiethanolamine, N-methyldiethanolamine, 1,10-decanediol, 1,12-dodecanediol and C36-diol (mixture of isomers of alcohols having the empirical formula $C_{36}H_{74}O_2$), solketal (the acetonide of glycerol), lactic acid, serine, hydroxymethylfuran, hydroxymethylfurfural, bis(hydroxymethyl)furan, glucose, lactose, maltose, mannose, fructose, isosorbide, isomannide, isoidide, sorbitol. It is possible to use all known triols. In order to obtain the diamines from hydroxymethylfurfural, bis(hydroxymethyl)furan, isosorbide, isomannide and isoidide, it is preferable, to use homogeneous catalysis. Examples of triols which can be used in the process of the invention are glycerol, trimethylolpropane and triethanolamine.

In the case of heterogeneously catalyzed amination processes, both in the gas and liquid phase, aromatic alcohols like phenol, dimethylphenol or mono- and dialkylphenols in general, are also suitable feedstocks.

Primary amines are compounds which have at least one primary amino group ($-NH_2$). Primary diamines have two primary amino groups. Primary triamines have three primary amino groups. Primary polyamines have more than three primary amino groups. Primary amines are valuable products with a large number of different uses, for example as solvents, stabilizers, for the synthesis of chelating agents, as starting materials for producing synthetic resins, inhibitors, interface-active substances, intermediates in the manufacture of fuel additives (U.S. Pat. No. 3,275,554 A, DE 2125039A and DE36 11 230 A), surfactants, drugs and crop protection agents, hardeners for epoxy resins, catalysts for polyurethanes, intermediates for producing quaternary ammonium compounds, plasticizers, corrosion inhibitors, synthetic resins, ion exchangers, textile auxiliaries, dyes, vulcanization accelerators and/or emulsifiers. Primary diamines and triamines are valuable products with a large number of different uses, for example as solvents, stabilizers, for the synthesis of chelating agents, as starting materials for producing synthetic resins, drugs, inhibitors, corrosion protectants, polyurethanes, as hardeners for epoxy resins, and interface-active substances.

Preferred amines that can be prepared according to the invention are selected from the group consisting of polyetheramines having a molecular weight of from 200 to 10000 g/mol and aliphatic, cycloaliphatic, heterocycloaliphatic and aromatic amines having from 2 to 20 carbon atoms and from 1 to 3 primary, secondary or tertiary amino groups. The amines can optionally contain further functional groups, in particular 1 to 3 hydroxyl groups.

Particularly preferred amines that can be prepared according to the invention are

(a) polyetheramines with varying molecular weight and number of functional groups, such as polyetheramine D230, D400, D2000, T403, T5000 and Jeffamine M2070;
(b) alkanolamines, such as 1,2- and 1,3-aminopropanol and aminoethanol;
(c) ethylene- and propyleneamines, such as ethylenediamine, diethylenetriamine, aminoethylethanolamine, 1,2-propylenediamine, piperazine and triethylenediamine (DABCO);
(d) alkyl-substituted ethyleneamines, such as N,N-diisopropylethylenediamine and N,N-diethylethylenediamine;
(e) alkylamines, such as ethylamines, propylamines, isopropylamines, butylamines, hexylamines, cyclohexylamines, octylamines, 2-ethylhexylamines, fatty amines and tridecylamines;
(f) cyclic alkylamines, such as pyrrolidine, piperidine and their alkylated derivatives;
(g) higher alkylene diamines, such as hexamethylenediamine, octamethylendiamine, nonanediamine and dodecanediamine;
(h) etheramines, such as aminodiglycol, diaminodiglycol and morpholine, N-methylmorpholine, methoxyisopropylamine and dimorpholinodiethyl ether (DMDEE);
(i) biobased amines, such as furfurylamine, tetrahydrofurfurylamine, aminopropanediol, isosorbide diamine, isomannide diamine, aminated sorbitol, bisaminomethylfurane and bisaminomethyltetrahydrofurane;
(k) aromatic amines, such as 2,6-dimethylaniline and 2-isopropyl-5-methylaniline.

The plural denotes all congeners, i.e., the mono, di and trialkylated amine of the respective hydrocarbon substituent. The preferred amines can be used as curing agents for epoxy composite materials, as polyurethane catalysts or building blocks for such catalysts, as neutralizing agents, feedstocks for detergents, auxiliaries and solvents in various kinds of production, e.g., the lyocell process, as building blocks for agrochemical and pharmaceutical active ingredients and the like.

The preferred amines can be obtained by the processes described above from common starting materials like polyetherols of varying molecular weight, architecture in terms of blocks of various alkyleneoxides and appropriate starters like dipropylene glycol, trimethylolpropane (TMP), pentaerythritol or end-capped ether alcohols; diols like monoethyleneglycol, diethyleneglycol (giving rise to morpholine, aminodiglycol and diaminodiglycol), propyleneglycol, 1,3-propanediol, butanediol (giving rise to pyrroldine), pentanediol (giving rise to piperidine) hexanediol, octanediol; simple naturally occurring alcohols like methanol, ethanol, higher alkanols like propanol, butanol, pentanol, hexanol, octanol; biobased alcohols like furfuryl alcohol, tetrahydrofurfuryl alcohol, glycerol and its acetonide, isosorbide, isomannide, sorbitol, bis(hydroxymethyl)furane, fatty alcohols and menthol; aromatic alcohols (phenols) like 2,6-dimethylphenol and thymol. Especially preferred are alcohols produced by fermentation or by chemical transformation from sugars, fats, biomass, wood and other renewable feedstocks or isolated from natural sources.

The inventive amine is characterized by a low molar share of deuterium in the hydrogen bound on the amine nitrogen and on carbon atoms adjacent (i.e., in alpha-position) to that amine nitrogen in the primary, secondary or tertiary amine group or groups formed in step (c) is $\leq$ 100 ppm, preferably in the range of from 10 to 95 ppm, more preferably in the range of from 15 to 90 ppm, most preferably in the range of from 20 to 80 ppm, especially in the range of from 30 to 75 ppm based on the total hydrogen content bound on the amine nitrogen and on carbon atoms adjacent (i. e., in alpha-position) to that amine nitrogen. Said amines are prepared by the process of the present invention comprising steps (a) to (c).

Said low deuterium molar share is introduced by step (a) of the process according to the present invention. Therefore, also the ammonia, prepared by reacting the hydrogen from step (a) with nitrogen in step (b) is characterized by this low deuterium molar share.

With the present invention environmentally friendly amines and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The

downstream products based on hydrogen, preferably amines and ammonia based on hydrogen obtained by electrolysis and hydrogen itself, can be distinguished by its deuterium molar share from amines, ammonia and hydrogen prepared by processes based on fossil energy, i.e. made by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are amines.

The present invention further relates to a process for tracing the origin, especially the energetic origin, of hydrogen bound in downstream compounds based on hydrogen by determining the molar share of deuterium in said downstream compounds based on hydrogen, wherein the downstream compounds are amines.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Suitable deuterium molar shares of the amines are mentioned in the present application.

The inventive processes of this application and the thus synthesized chemical compounds can be used in the synthesis of surfactants: Alcohols and compounds having single or multiple hydroxy-groups, including sugar alcohols, can be converted to the corresponding amines using the inventive hydrogen. Suitable aminating agents in the amination of alcohols under hydrogenating conditions are both ammonia and primary or secondary amines. Making amines from alcohols using the inventive hydrogen can for example be carried out by the methods described in US5166433A and US20100311973A1 and the references cited therein. Examples for the conversions described above are given below. Such Amines can be used to produce surface active compounds, for example surfactants, for example by converting the amino group to the corresponding amine oxide or by alkoxylation. Preferred amine oxides are alkyl dimethyl amine oxides or alkyl amido propyl dimethyl amine oxides, more preferably alkyl dimethyl amine oxides and especially coco dimethyl amino oxides. Amine oxides may have a linear or mid-branched alkyl moiety. Another example of surfactants that can be produced from the amines described above are quaternary ammonium surfactants, which can have up to 26 carbon atoms and include: alkoxylated quaternary ammonium (AQA) surfactants as discussed in US 6,136,769; dimethyl hydroxyethyl quaternary ammonium as discussed in US 6,004,922; dimethyl hydroxyethyl lauryl ammonium chloride; polyamine cationic surfactants as discussed in WO 98/35002, WO 98/35003, WO 98/35004, WO 98/35005, and WO 98/35006.

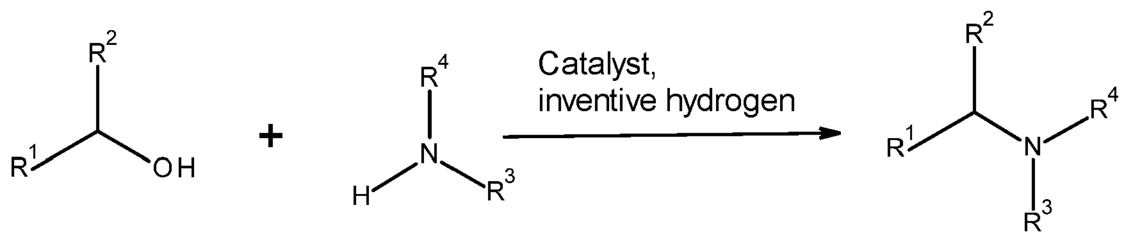


$R^1$ is an organic moiety, $R^2$ to $R^4$ can be organic moieties or hydrogen

Examples for using the reactive hydrogen to converting alcohols into amines that can be used to manufacture surface active compounds are given in the table below.

| $R^1$ | $R^2$ | $R^3$ | $R^4$ | Conditions |
|---|---|---|---|---|
| n-$C_{11}H_{23}$ | H | $CH_3$ | H | 30 bar $H_2$, 180 °C, Catalyst A (described in US5166433A) |
| Iso-$C_8H_{17}$ | H | H | H | 30 bar $H_2$, 180 °C, Catalyst A (described in US5166433A) |
| 4-$C_9H_{19}$ | H | H | H | 30 bar $H_2$, 180 °C, Catalyst A (described in US5166433A) |

Furthermore, the products according to the process described herein can be converted to a product as described on page 18, line 29 to page 26, line 29 of European patent application No. EP24164893.0.

Examples

I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell:_Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nation® 117 standard membrane (supplier DuPont, dry thickness 180 microns) was assembled by HIAT GmbH with catalytic active coating materials iridium (19 g/$m^2$) and platinum (8 g/$m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell:_ Alkaline water electrolysis was conducted with a circular AEC electrolysis cell (model Electro MP Cell, supplier ElectroCell Europe A/S, 0.01 $m^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell:_The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station: As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.

The hydrogen stream was used with no further purification/dryer. Remaining gas moisture is separated by

desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

The results of the hydrogen production are shown in Table 1.

Table 1

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |

* Isar river surface water (Munich) collected in winter season January 2021.

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples (ammonia, amines and alcohols) was executed via isotope ratio monitoring gas chromatography/mass spectrometry (IRMS). Therefore, a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^2$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Production of Amines

Amines are prepared with catalytic synthesis processes in industrial scale as described above.

By using non-fossil-based energy for electrolytic hydrogen in both the ammonia and the hydrogen synthesis for the reductive amination of aldehydes and ketones, a substantial fraction of the hydrogen is originating from the electrolytic hydrogen with a low deuterium content. Especially for ammonia this is a big difference since the fossil-based production process relies on synthesis gas made from fossil natural gas and additionally large quantities of steam. This steam reforming step is not required for the non-fossil ammonia route based on pure hydrogen and nitrogen.

In all of the large-scale commercial production processes in question using non-fossil hydrogen no significant additional amounts of hydrogen-species are introduced in the "input/output" process material balance. This is required in order to minimize output of undesired wastewater or other liquid and gaseous emission streams and to ensure high yields

with purity according to the product specification.

In case water is used as processing aid for stripping/scrubbing/cleaning or quenching/condensing of process-internal streams in e.g. the ammonia or amines synthesis steps this water is always run in an almost completely closed cycle with minimum purge ensuring insignificant cross-contamination with deuterium.

## Claims

**1.** A process for the preparation of amines comprising the following steps:

(a) providing hydrogen with a molar share of deuterium ≤ 100 ppm, based on the total hydrogen content, by electrolysis of water using electrical power generated at least in part from non-fossil energy,
(b) reacting the hydrogen from step (a) with nitrogen to form ammonia,
(c) reacting the ammonia from step (b) with an alcohol R-OH in the presence of hydrogen from step (a) to form the corresponding primary, secondary and/or tertiary amines $R\text{-}NH_2$, $R_2NH$ and/or $R_3N$.

**2.** The process of claim 1, wherein the electrical power is generated from wind power, solar energy, biomass, hydropower and geothermal energy.

**3.** The process according to claim 1 or 2, wherein hydrogen is provided by polymer electrolyte membrane water electrolysis.

**4.** The process according to claim 3, wherein hydrogen is provided by proton exchange membrane water electrolysis (PEMWE) or anion exchange membrane water electrolysis (AEMWE).

**5.** The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

**6.** The process according to any one of claims 1 to 5, wherein step (c) is carried out as heterogeneously or homogeneously catalyzed reaction.

**7.** Amines, obtainable in the process according to any one of steps 1 to 6.

**8.** Amines according to claim 7, wherein the molar share of deuterium in the hydrogen bound on the amine nitrogen and on carbon atoms adjacent to that amine nitrogen in the primary, secondary or tertiary amine group or groups formed in step (c) is ≤ 100 ppm, preferably in the range of from 10 to 95 ppm, more preferably in the range of from 15 to 90 ppm, most preferably in the range of from 20 to 80 ppm, especially in the range of from 30 to 75 ppm based on the total hydrogen content bound on the amine nitrogen and on carbon atoms adjacent to that amine nitrogen.

**9.** Amines according to claim 7 or 8, selected from the group consisting of polyetheramines having a molecular weight of from 200 to 10 000 g/mol and aliphatic, cycloaliphatic, heterocycloaliphatic and aromatic amines having from 2 to 20 carbon atoms and from 1 to 3 primary, secondary or tertiary amino groups.

**10.** Amines according to any one of claims 7 to 9, selected from the group consisting of

(a) polyetheramines with varying molecular weight and number of functional groups, such as polyetheramine D230, D400, D2000, T403, T5000 and Jeffamine M2070;
(b) alkanolamines, such as 1,2- and 1,3-aminopropanol and aminoethanol;
(c) ethylene- and propyleneamines, such as ethylenediamine, diethylenetriamine, aminoethylethanolamine, 1,2-propylenediamine, piperazine and triethylenediamine (DABCO);
(d) alkyl-substituted ethyleneamines, such as N,N-diisopropylethylenediamine and N,N-diethylethylenediamine;
(e) alkylamines, such as ethylamines, propylamines, isopropylamines, butylamines, hexylamines, cyclohexylamines, octylamines, 2-ethylhexylamines, fatty amines and tridecylamines;
(f) cyclic alkylamines, such as pyrrolidine, piperidine and their alkylated derivatives;
(g) higher alkylene diamines, such as hexamethylenediamine, octamethylendiamine, nonanediamine and dodecanediamine;
(h) etheramines, such as aminodiglycol, diaminodiglycol and morpholine, N-methylmorpholine, methoxyisopropylamine and dimorpholinodiethyl ether (DMDEE);
(i) biobased amines, such as furfurylamine, tetrahydrofurfurylamine, aminopropanediol, isosorbide diamine, isomannide diamine, aminated sorbitol, bisaminomethylfurane and bisaminomethyltetrahydrofurane, 2-octylamine;
(k) aromatic amines such as 2,6-dimethylaniline and 2-isopropyl-5-methylaniline.

**11.** The use of the molar share of deuterium in downstream compounds based on hydrogen for tracing the origin, especially the energetic origin, of hydrogen bound in the downstream compounds based on hydrogen, wherein the downstream compounds are amines.

**12.** The use according to claim 11, wherein the downstream compounds are amines according to any one of claims 7 to 10.

**13.** A process for tracing the origin, especially the energetic origin, of hydrogen bound in downstream compounds based on hydrogen by determining the molar share of deuterium in said downstream compounds based on hydrogen, wherein the downstream compounds are amines.

**14.** The process according to claim 13, wherein the downstream compounds are amines according to any one of claims 7 to 10.

Europäisches Patentamt
European Patent Office
Office européen des brevets

# EUROPEAN SEARCH REPORT

Application Number
EP 24 20 7994

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/136097 A1 (SMAJLOVIC IVAN [RS]) 9 June 2011 (2011-06-09)<br>* the whole document *<br>----- | 11-14 | INV.<br>C25B1/04<br>C25B15/08<br>C01B4/00<br>C01C1/04<br>C07B59/00<br>C07C209/14 |
| A | FELLOWS S K ET AL: "Availability of large quantities of low-deuterium hydrogen, and possible uses", INTERNATIONAL JOURNAL OF HYDROGEN ENERGY, ELSEVIER, AMSTERDAM, NL, vol. 6, no. 1, 1 January 1981 (1981-01-01), pages 67-71, XP025591252, ISSN: 0360-3199, DOI: 10.1016/0360-3199(81)90098-7 [retrieved on 1981-01-01]<br>* the whole document *<br>----- | 11-14 | |
| Y | US 2020/148547 A1 (PAPILE CHRISTOPHER [US]) 14 May 2020 (2020-05-14)<br>* the whole document *<br>----- | 1-14 | |
| Y | TOMAS TLUSTY ET AL: "Gas phase amination of octan-1-ol over a cu-cr catalyst", REACTION KINETICS AND CATALYSIS LETTERS, SPRINGER SCIENCE+BUSINESS MEDIA, DORDRECHT, NL, vol. 88, no. 2, 1 August 2006 (2006-08-01), pages 371-379, XP019406399, ISSN: 1588-2837, DOI: 10.1007/S11144-006-0074-7<br>* the whole document *<br>----- | 1-14 | TECHNICAL FIELDS SEARCHED (IPC)<br>C25B<br>C01C<br>C07C<br>C07B<br>C01B |
| Y | US 2007/149818 A1 (FUKUSHIMA TETSUAKI [JP] ET AL) 28 June 2007 (2007-06-28)<br>* the whole document *<br>----- | 1-14 | |
| A,P | WO 2023/213713 A1 (BASF SE [DE]) 9 November 2023 (2023-11-09)<br>* the whole document *<br>----- | 11-14 | |

The present search report has been drawn up for all claims

1

| Place of search | Date of completion of the search | Examiner |
|---|---|---|
| Munich | 5 March 2025 | Ritter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 20 7994

This annex lists the patent family members relating to the patent documents cited in the above-mentioned European search report. The members are as contained in the European Patent Office EDP file on
The European Patent Office is in no way liable for these particulars which are merely given for the purpose of information.

05-03-2025

| Patent document cited in search report | Publication date | Patent family member(s) | Publication date |
|---|---|---|---|
| US 2011136097 A1 | 09-06-2011 | NONE | |
| US 2020148547 A1 | 14-05-2020 | NONE | |
| US 2007149818 A1 | 28-06-2007 | CN 1990454 A | 04-07-2007 |
| | | DE 102006061045 A1 | 26-07-2007 |
| | | JP 4989888 B2 | 01-08-2012 |
| | | JP 2007176889 A | 12-07-2007 |
| | | US 2007149818 A1 | 28-06-2007 |
| WO 2023213713 A1 | 09-11-2023 | AU 2023266064 A1 | 14-11-2024 |
| | | CN 119156370 A | 17-12-2024 |
| | | EP 4519241 A1 | 12-03-2025 |
| | | KR 20250006960 A | 13-01-2025 |
| | | WO 2023213713 A1 | 09-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014016241 A
- WO 2012119927 A
- WO 2012119929 A
- WO 2012113475 A
- EP 1312600 A
- EP 1312599 A
- US 3275554 A
- DE 2125039 A
- DE 3611230 A
- US 5166433 A
- US 20100311973 A1
- US 6136769 A
- US 6004922 A
- WO 9835002 A
- WO 9835003 A
- WO 9835004 A
- WO 9835005 A
- WO 9835006 A
- EP 24164893


### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31 395
- **H. SATO et al.** *International Journal of Hydrogen Energy*, 2021, vol. 46, 33 689-33 695
- **K.S. HAYES**. *Applied Catalysis A: General*, 2001, vol. 221, 187-195
- Amines, Aliphatic. *Ullmann's Encyclopedia of Industrial Chemistry*, 6-7
- **C. YUE et al.** *Arabian Journal of Chemistry*, 2022, vol. 15, 103865
- **B. MORANDI et al.** *Synthesis*, 2017, vol. 49, 776-789
- **Q. YANG et al.** *in Chem. Soc. Rev.*, 2015, vol. 44, 2305-2329
- **S. BÄHN et al.** *in ChemCatChem*, 2011, vol. 3, 1853-1864
- Ullmann's Encyclopedia of Industrial Chemistry, vol. 4, 199-238
- Reactor Types and Their Industrial Applications. **K.D. HENKEL**. Ullmann's Encyclopedia of Industrial Chemistry. Wiley VCH Verlag GmbH & Co. KGaA, 2005
- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionization mass spectrometry (TIMS). *Vacuum*, 2014, vol. 101, 416-422
- RAPID COMMUNICATIONS IN MASS SPECTROMETRY. *Rapid Commun. Mass Spectrom.*, 1999, vol. 13, 1226-1230
- **W.A. BRANDT et al.** *Sensitivity is within +/-3 ppm.*
- SNIF-NMR Part 1: Principles. **GÉRARD J. MARTIN** ; **SERGE AKOKA** ; **MARYVONNE L. MARTIN**. Modern Magnetic Resonance. Springer, 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008, 1669-1680